# EUROPEAN PATENT APPLICATION

(11) **EP 1 552 838 A1**
(43) Date of publication of application: **13.07.2005**
(21) Application number: 03733206.1
(22) Date of filing: 30.05.2003
(51) Int. Cl.: A61K 31/7048, A61K 31/198, A61K 31/375, A61K 35/20, A61K 35/72, A61K 35/78, A61P 1/16, A61P 17/16, A61P 21/00, A61P 25/28, A61P 27/02, A61P 35/00, A61P 37/04, A61P 39/00, A61P 43/00, C07H 17/04

(54) **RUBROFUSARIN GLYCOSIDE-CONTAINING COMPOSITION**

(30) Priority: 31.05.2002 JP 2002160176
(71) Applicant: SUNTORY LIMITED, Osaka-shi, Osaka 530-8203 (JP)
(72) Inventor: ASAMI, Sumio, Ibaraki-shi, Osaka 567-0886 (JP)
(74) Representative: VOSSIUS & PARTNER
(86) International application number: PCT/JP2003/006875
(87) International publication number: WO 2003/101466

(57) **Abstract**

There is provided an oral composition capable of strimulating intracellular biosynthesis of glutathione in order to maintain in-vivo (i.e., in tissues and cells) glutathione level at a high level, by adding an active ingredient other than an amino acid precursor from a viewpoint of increasing intracellular glutathione synthesis, as a method which is an alternative or can be combined with a method of supplying an amino acid precursor to be used in glutathione biosynthesis. The oral composition is used for maintaining or increasing intracellular glutathione level and contains at least one rubrofusarin glycoside in an amount that is effective for increasing the intracellular glutathione.

## Description

### TECHNICAL FIELD

The present invention relates to an oral composition containing rubrofusarin glycoside as an active ingredient, the composition being capable of increasing an in-vivo glutathione level.

### BACKGROUND ART

Glutathione is a tripeptide composed of glutamic acid, cysteine, glycine and a polyfunctional biological substance contained at a high concentration of 1 to 10 mM within cells. Glutathione has antioxidative activity. Besides this, it serves as a substrate for glutathione peroxidase, and thus plays an important role in eliminating hydrogen peroxide and lipid peroxide, and it further serves as a substrate for the xenobiotic metabolizing enzyme, glutathione-S-transferase, and thus takes part in detoxication metabolism. Glutathione is also used as a substrate for glutathione dehydrogenase for reactivating dehydroascorbic acid. Further, in recent years, it has been revealed that an in-vivo signal transfer varies depending on an intracellular redox state, which suggests that glutathione present in a high concentration plays a role as an intracellular redox buffer (General remarks: Ann, Rev. Biochem. 52, 711, 1983, Curr. Top. Cell, Regul., 36, 1, 2000, Curr. Top, Cell, Regul. 36, 151, 2000).

As the population rapidly ages, serious concerns have arisen about increases in lifestyle-related diseases represented by diabetes, its prodromal states, and brain disorders such as Parkinson's disease and Alzheimer's disease. It has been elucidated that in-vivo oxidative stress is deeply involved in these morbid states. To evaluate such oxidative stress, a decrease in tissue glutathione level is often measured and used as an indicator. As described, since a decrease in tissue/intracellular glutathione level is observed in many disorders due to oxidative stress, as well as to senescence with aging, it is considered that an enhancement of in-vivo glutathione level will be significantly effective from a prophylactic point of view (general remarks: Ann, Pharmacother, 29, 1263, 1995, Alt. Med. Rev. 2, 155, 1997, Free Rad, Biol, Med. 28, 1405, 2000, Prog. Neurobiol. 62, 649, 2000).

As a method of increasing in vivo glutathione level, oral intake of glutathione is readily apparent. However, glutathione is rapidly decomposed by a glutathione-decomposing enzyme (transpeptidase) in the digestive tract and the liver. Even if a large amount of glutathione is taken, blood glutathione level does not increase. From these facts, it is known that oral intake of glutathione does not directly contribute to increasing tissue glutathione level (Eur. J. Clin. Pharmacol. 43, 667, 1992).

Therefore, since it is pointed out that administration of glutathione itself is not effective in increasing tissue glutathione level due to a half-life in blood which is as short as only several minutes, attempts to develop pharmaceutical agents have been made to solve this problem.

As a compound for promoting biosynthesis of endogenous glutathione, WO94/12527 discloses an acyl derivative of γ-L-pyroglutamyl-L-cysteine, which is effective for treating various diseases caused by a decrease or deficit of glutathione, such as morbid states involved in oxidative tissue damages and especially damage caused by excessive free radials. Examples of such diseases mentioned herein include intracellular oxidation states caused by drinking excessive amounts of alcohol, xenobiotic substances, X-ray damage, hepatopathy, toxic effects caused by pharmaceutical drugs or compounds, toxic effects caused by heavy metals, physiological aging of the brain (e.g., memory loss and loss of learning ability, Parkinson's degeneration, which is degeneration of the brain caused by a decrease of glutathione level involved in a change in protection mechanism against in-vivo oxidative substances), acute or chronic neurologic diseases (as acute disorders, mention is made of brain attack being an acute ischemic state, low blood sugar level, and epileptic stroke; and as chronic disorders, mention is made of amyotrophic lateral sclerosis, Alzheimer's disease, and Huntington's disease, etc.), and dysfunction of immune mechanism, in particular, a decrease in immunological protection against cancer, and infertility, in particular, male infertility. Furthermore, WO 94/12527 discloses that the aforementioned compound is also suitable for treating ischemia/reperfusion disorders of organs which are main causes for generating free radicals.

Furthermore, Japanese Patent Laid-Open No. 1-26516 discloses γ-L-glutamyl-L-cysteine methyl ester serving as a compound capable of increasing glutathione level which is effective for treating or preventing diseases including cataract, hepatopathy and nephropathy.

Moreover, S-lower fatty acid glutathione derivatives are disclosed as an anti-inflammatory agent, an anti-allergic agent, or a hepatopathy preventing agent (WO91/12262, WO91/16337), and sugar-containing glutathione derivatives are disclosed as a liver-protecting agent (Japanese Patent Laid-Open No. 9-25292), which suggests that a wide variety of diseases can be effectively treated and prevented by increasing tissue glutathione.

It has been reported that glutathione ester derivatives have protective effects against tissue/cell damages as follows: buthionine sulfoximine-induced cataract (Pro. Natl, Acad. Sci. 86, 8727,1988); acetaminophen-induced hepatopathy (Biochem. Pharmacol, 39, 1877, 1990); damage by anti-cancer agent such as Cisplatin and alkylating agents (FASEB J. 4, 3251, 1990, Cancer 62, 1275, 1988); cytotoxicity by ultraviolet-B (UV-B) Radiation(Biol. Pham. Bull. 18, 1219, 1995); t-butyl hydroperoxide-induced stomach cell damage (Eur. J. Phamacol, 351, 363, 1998); and liver ischemic reperfusion injury (J. Surg. Res. 86, 2, 1999). Furthermore, as recent findings on L-2-oxothiazolidine-4-carboxylic acid, which is a prodrug of cysteine and increases tissue glutathione level, mention is made of improvement of cerulein-induced pancreatitis (Gastroenterology 112, 1681, 1997) and vascular endothelial function of coronary arteriasis patients (J. Clin. Invest. 101, 1408, 1998), prevention of endotoxin-induced cardiac dysfunction (Am. J. Respir. Crit. Care Med. 158, 1109, 1998), prevention of chronic alcohol hepatopathy (Hepatology 31, 391, 2000), and mitigation of deuteropathy involved in spinal injury (FASEB J. 15, 243, 2001).

It should be noted that such attempts to treat disorders are for medical purposes and are directed to medical care in emergency or serious cases. From a view point of daily health-care for improving quality of life (QOL), it has been expected to develop a means for returning a reduced tissue glutathione level to normal in consideration of the fact that glutathione is a biological substance that is "synthesized and supplied by a living body itself."

### DISCLOSURE OF THE INVENTION

The present invention provides an oral composition capable of stimulating intracellular biosynthesis of glutathione in order to maintain in-vivo (i.e., in tissues and cells) glutathione level to be high, wherein the composition comprises an active ingredient other than an amino acid precursor from a viewpoint of increasing intracellular glutathione synthesis. The composition provides a method which can be replace or be combined with a method of supplying an amino acid precursor for glutathione biosynthesis.

### BRIEF DESCRIPTION OF THE DRAWINGS

Figure 1 is a graph showing that the intracellular glutathione level of HepG2 cells derived from human hepatic cells increases in a time-dependent manner by rubrofusarin glycoside in comparison with silibinin.
Figure 2 is a graph showing that the intracellular glutathione level of HepG2 cells derived from human hepatic cells increases in a dose-dependent manner by rubrofusarin glycoside in comparison with β-naphthoflavone and silibinin.
Figure 3 is a graph showing an effect of rubrofusarin glycoside in protecting HepG2 cells derived from human hepatic cells from being killed by t-butyl hydroperoxide, in comparison with β- naphthoflavone used as a positive control.
Figure 4 is a graph showing rubrofusarin glycoside and cystine, which is a precursor amino acid of glutathione, synergistically contribute to an increase of the intracellular glutathione level of HepG2 cells derived from human hepatic cells.
Figure 5 is a graph showing that oral intake of a concentrate containing rubrofusarin glycoside exhibits suppressive effects against the reduction of glutathione level in the hepatic tissue and the increase of lipid peroxidation (TBARS value) caused by restraint stress.
   A: Glutathione content in the liver
   B: TBARS value of the liver
Figure 6 is a graph showing that oral intake of a concentrate containing rubrofusarin glycoside exhibits inhibitory effects against the reduction of glutathione level in the brain tissue and the increase of lipid peroxidation (TBARS value) caused by restraint stress.
   A: Glutathione content in the brain tissue
   B: TBARS value of the brain tissue

### DETAILED DESCRIPTION OF THE INVENTION

The present inventors investigated food materials widely in use with a view to finding an active ingredient capable of increasing the ability of cells to synthesize glutathione and suitable for solving the abovementioned problem. As a result, they found that a Cassia obtusifolia extract contains an active ingredient identified as rubrofusarin glycoside. As substances useful for increasing intracellular glutathione level, synthetic compounds such as β-naphthoflavone (J. Biol. Chem, (1998) 273, 14683)) and naturally occurring compounds such as silimarin (Planta Med. 55, 420, 1989), cyano hydroxy butene (Toxicol. Appl. Pharmacol/ 123, 257, 1993), apocynin (FEBS Lett, 443, 235, 1999) are already known; however, the effect of rubrofusarin glycoside in increasing intracellular glutathione level has until now not been known.

It should be noted that Cassia seed is the seed of Cassia obtusifolia (Japanese name: ebisugusa) belonging to the leguminous family. Cassia seed has traditionally been used by being roasted and brewed. According to "Chuyaku Dai-jiten" (published by Shanghai Science Technology publisher, edited by Shogakukan Inc.), there is a description reading "Cassia seed has efficacy in cleansing the liver, making eyes clear, being good for diuresis, and causing bowel movement", and in particular, it is described that Cassia seed is a herbal medicine effective for eyes, by referring to the teaching of a sacred script (Shin Nouhon Soukei) that "(Cassia seed) cures acyanopsia, edematous eye, hemorrhagic conjunctivitis, red eyes, eye pain, and incessant tearing, and long term use will be beneficial for eyelight". In the book "Chinese Medicine for Clinical Cases" (Ishiyaku Publishers, Inc.), Section "Cassia seed", there is a description reading: "Cassia seed is referred to as a general medicine for eyes". However, the mechanism underlying the efficacy of Cassia seed on eyes is unknown. On the other hand, glutathione is known to be accumulated in a significant amount in the ocular tissue including the crystalline lens and has a role to play in these tissues. Further, glutathione is used as an ophthalmological drug (therapeutic agent for cataract) for treating early-stage senile cataract, corneal ulcer, ectocorneal exfoliation, and keratitis (refer to Regular Medicine Information Miscellany for Pharmacists, published by Hirokawa Shoten). Taking the above- mentioned knowledge into consideration in combination with the findings of the present invention, it is considered that the medical effect of Cassia seed resides in that rubrofusarin glycoside contained in Cassia seed increases the intracellular glutathione level of the ocular tissue, thereby maintaining and improving the function of eyes. Neither the mechanism of the efficacy of Cassia seed nor the active ingredient in Cassia seed was known prior to the present invention.

The present invention is directed to an oral composition containing rubrofusarin glycoside as an active ingredient for maintaining in-vivo (in tissue and cells) glutathione level at a high level.

Rubrofusarin glycoside is represented by the following formula: 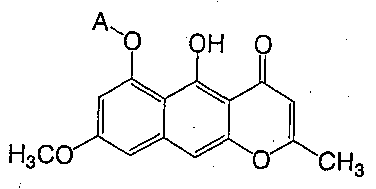 where, A represents a sugar component.

Examples of sugar component A include gentiobiose and glucose and the like. A preferable sugar component is gentiobiose. In that case, the rubrofusarin glycoside is rubrofusarin gentiobioside.

Rubrofusarin glycoside is contained in an extract derived from naturally occurring substances. Examples of such naturally occurring substances are Cassia obtusifolia and Cassia occidentalis belonging to the Cassia genus.

Rubrofusarin glycoside may be used as an active ingredient of the composition of the present invention in a pure form or in a crude product extracted from a naturally occurring substance. Preferable a naturally occurring substance to be used as a raw material is Cassia seed, but is not limited thereto. Any substance may be used as long as it contains rubrofusarin glycoside, and hence, Cassia occidentalis and others may be similarly used.

When rubrofusarin glycoside is to be taken in the form of Cassia seed itself for attaining the object of the present invention, Cassia seed must be brewed and taken in a dose of 5 to 30 g per day. However, in view of current life styles, it is not easy to continue taking such a medication. Furthermore, people tend to avoid taking brewed Cassia seed because of its unpleasant odor, and harsh and bitter taste. Although, some recent commercially available blend teas contain roasted Cassia seed as senna tea for the purpose of imparting flavor and taste, the amount of rubrofusarin glycoside in such teas is significantly low, and such preparations remain far from satisfying the object of the present invention. This is because when dry Cassia seed is roasted, the content of rubrofusarin glycoside significantly decreases (see Example 3).

Therefore, it is preferred to use a concentrate composition containing a predetermined amount of rubrofusarin glycoside, which is obtained by subjecting a raw material, dry Cassia seed containing rubrofusarin glycoside, to appropriate treatments including extraction.

As solvents useful for extracting rubrofusarin glycoside from a naturally occurring raw material, water or an organic solvent can be mentioned. Examples of an organic solvent include alcohols such as ethanol and methanol, esters such as ethyl acetate, ketones such as acetone, and ethers such as methylethyl ether. It is also possible to use two or more selected from water and these organic solvents in combination. To produce foods and drinks, water or an aqueous ethanol solution is appropriate.

As an example of preparing the extraction concentrate containing rubrofusarin glycoside according to the present invention by using Cassia seed as a raw material, the following method may be used. In this case, a method and conditions capable of providing as high a level of rubrofusarin glycoside as possible in the concentrate should be employed. For example, the concentrate desirably contains rubrofusarin glycoside in a ratio of 1 to 30 wt% based on the concentrate (dry amount). A solvent is directly added to Cassia seed or it is added after Cassia seed is pulverized by a conventional method in order to increase extraction efficiency. Thereafter, extraction is performed for a time of from about 30 minutes to 4 hours under warming or for 1 to 3 days at room temperature. The extraction solution is filtered to obtain a crude extraction filtrate. Warming is preferably performed at a temperature of 50 to 95°C. The volume of a solvent for use in extraction may be, for example, 3 to 10 fold that of the raw material.

The content and purity of rubrofusarin glycoside extracted and contained in the filtrate will be about 2.2% by extraction with 70% ethanol and about 1.1% by extraction under heating (see Table 1). However, there are some cases where it is desirable to further improve purity. For this purpose, for example, an adsorbent resin such as Diaion HP20 (Mitsubishi Chemical Corporation) generally used industrially and activated charcoal may be used. When rubrofusarin glycoside is extracted with water, a filtrate of the extract is brought into direct contact with the resin. When rubrofusarin glycoside is extracted with an aqueous ethanol solution as a solvent, the ethanol concentration is reduced under a vacuum or the like and the resultant extract is brought into contact with the resin. This reduction of the ethanol concentration is preferred since it facilitates sufficient adsorption of rubrofusarin glycoside. Specifically, it is preferred to reduce the concentration of ethanol to 20% or less.

When the active ingredient is eluted from the resin, a desired concentration (% by weight) of the active ingredient is first selected depending upon the purpose, and a suitable method to provide the concentration may be chosen. For example, in the case of an ethanol solution, the extraction may be performed by a stepwise elution or concentration gradient method. A stepwise elution can be performed with, but is not limited to, 20% ethanol wash/80% ethanol elution or 30% ethanol wash/60% ethanol elution.

The eluate thus obtained can be concentrated or formed into dry powder by a method such as conventional vacuum concentration and lyophilization. In a case that dry powder is not easily obtained from the concentrate, dextrin, a high molecular weight starch hydrolysis product, or a high molecular weight peptide preparation can be used as a conventional excipient to produce a dry powder.

To prepare a further purified rubrofusarin glycoside preparation (for example, highly purified rubrofusarin gentiobioside), the extraction concentrate obtained above may be subjected to a conventional process such as column chromatography, high-performance liquid column chromatography, or liquid-liquid distribution chromatography to obtain a product of a desired purity. For example, when elution is performed by reverse-phase chromatography using an aqueous acetonitrile solution comprising a concentration gradient of acetonitrile from 10% to 80% in 0.1% trifluoroacetic acid as a solvent, an active ingredient can be eluted as a finely separated peak with a good reproducibility. From the fraction of the peak, rubrofusarin glycoside (for example, rubrofusarin gentiobioside) can be obtained substantially as a single ingredient.

To monitor the purity and yield of rubrofusarin glycoside in the extraction and purification process mentioned above, the assay method described in Example 2 may be used.

Rubrofusarin glycoside is an ingredient contained in an amount of 3 mg or more per 1 g of fresh Cassia seed, as listed in Table 1 shown later. Rubrofusarin glycoside has traditionally been taken as a conventional drink in a dose of 5 to 30 g per day in terms of fresh Cassia seed as Cassia brewed tea or senna tea. Since rubrofusarin glycoside has been orally taken as mentioned above, the safety of rubrofusarin glycoside is considered to be high. These factors must be taken into consideration in deciding the amount of rubrofusarin glycoside in the present invention. Therefore, in the present invention, the content of rubrofusarin glycoside serving as the active ingredient in the composition substantially has no upper limit; however, rubrofusarin glycoside is preferably contained in an amount of 0.5 wt% to 30 wt%, more preferably, 1 wt% to 15 wt% of the solid matter.

It is possible to provide an oral composition from which one can intake an effective amount of rubrofusarin glycoside, by admixing it in the composition as the pure substance or as a concentrate derived from a naturally occurring raw material. Rubrofusarin glycoside has sufficiently high stability to pH, water content, oxidation, light and the like and its taste is somewhat bitter. Consequently, the oral composition of the present invention can be provided in various forms such as foods and drinks, seasonings, alcohol beverages, functional foods, and medicinal products and is capable of preventing a decrease of glutathione level within cells, tissues and organs induced by oxidative stress or of maintaining the glutathione level. For example, rubrofusarin glycoside is effective for maintaining and improving functions of the liver, brain, and vision, and immune system; mitigating fatigue; suppressing damage of organs involved in enhanced detoxication metabolism; reducing risk of cancer; maintaining and activating the immune system ; recovering from muscle fatigue; preventing dullness of the skin; preventing lifestyle-related diseases; preventing smoking induced disorders; treating chronic fatigue syndrome due to AIDS; and preventing aging, all of which are related to oxidative stress.

As specific forms of the composition, mention may be made of a solid form, semi-fluid form, and fluid form. Examples of solid-forms of foods include general foods and health foods such as in biscuit-form, sheet-form, tablet-form (such as capsules and tablets) and grain powder-form. Examples of semi-fluid forms of foods include general foods and health foods in paste-form, jelly-form, and gel-form. Examples of fluid forms of foods include general foods and health foods in juice-form, cold beverage-form, tea-form, and health drink-form.

The composition of the present invention is not particularly limited except that it contains rubrofusarin glycoside in such an amount that plays a role in increasing intracellular glutathione level, and is safe to be taken orally.

Furthermore, rubrofusarin glycoside may be contained either as a single active ingredient in the foods or drinks as mentioned above or in combination with a precursor of glutathione for intracellular biosynthesis, in particular, L-cysteine/L-cystine, which is a nutritionally rate-determining amino acid, (however, free L-cysteine is toxic if orally administered in a significant amount as is known from Am. J. Clin. Nutr. 50, 1401, 1989) and L-methionine with a view to achieving a synergistic glutathione increasing effect. The "synergistic increase" means that when the sum of the intracellular glutathione level increasing effect by a composition containing rubrofusarin glycoside alone and that by a composition containing a precursor alone is compared to the increasing effect by a composition containing both ingredients at the same time, the latter effect is greater than the former effect. Examples of precursors include animal proteins such as egg white, egg membrane and milk whey rich in L-cysteine/L-cystine and L-methionine; vegetable proteins such as soy bean; proteins derived from yeast and the like; or peptides processed and prepared from these proteins; or approved food additives such as L-cystine, and L-methionine. It is preferable that these precursors or food containing the precursors in a large amount may contain L-cysteine in the composition in an amount of about 0.1 to 10 wt% on a solid basis.

Furthermore, to maintain intracellular glutathione level and allow glutathione to act efficiently, it will be preferred not only to stimutate biosynthesis of glutathione from its precursor but also to supply vitamin C (ascorbic acid) which is capable of reducing glutathione consumption.

The oral composition is effective for mitigating the aforementioned defective physical conditioning brought about by life style. The oral composition may be taken before and after a meal, during a meal or between meals, and particularly desirably between meals. The oral composition may be taken desirably at a frequency of 3 times per day, but is not limited to this and may be taken at any time. The tablet shown in Example 10 is designed in accordance with such an intake plan. The oral composition of the present invention is particularly and desirably taken by persons who are concerned about bloodshot eyes and decreased vision, persons who are at risk of senile cataract due to aging, persons who are at risk of the affects of smoking, and persons who are at risk of rough skin, wrinkles, and spots due to smoking, ultraviolet rays including sunlight, and aging.

### Example 1: Identification of active ingredient

An 70% (v/v) ethanol extract of Cassia seed was loaded on Diaion HP20 adsorbent column, washed with a 30% ethanol solution and eluted with a 70% ethanol solution. The resultant extraction concentrate was further purified by reverse-phase high-performance liquid chromatography to identify the active ingredient of the present invention.

Reverse-phase high performance liquid chromatography was performed by using a develosil ODS-HG5 column (6 × 100 mm, manufactured by Nomura Chemical Co., Ltd.) on a device equipped with a three dimensional monitor. 10 µl of the extraction concentrate (89 mg/ml aqueous solution) was injected and elution was carried out by a linear gradient of acetonitrile in 0.1% trifluoroacetic acid changing from 25% to 40% in 12 minutes. Fractions were taken at 15-second intervals, dried under reduced pressure, and thereafter dissolved in 20 µl of dimethylsulfoxide (DMSO).

An aliquot of 6 µl was taken from the DMSO solution of each of the factions and diluted with 194 µl of RPMI1640 medium (supplemented with 10% serum), and thereafter, subjected to a serial two-fold dilution in RPMI1640 medium to obtain 4 samples in different dilution stages and then the activity of each sample to increase intracellular glutathione level was determined.

The activity to increase intracellular glutathione level was determined by use of human hepatoma HepG2 cells. More specifically, HepG2 cells were cultured in RPMI1640 medium and added to a 96-well microplate at a density of 2.5 × 10⁴ cells (100 µl) per well. After the HepG2 cells settled on the wall, 25µl of each of the aforementioned samples was added and the mixture was cultured in a CO₂ incubator for 48 hours. Thereafter, a culture solution was removed and the cells were washed once with phosphate buffered saline (PBS), 25 µl of a PBS solution containing 0.25 mM monochlorobimane was added and the mixture was allowed to stand in the CO₂ incubator (37°C) for 45 minutes. After the mixture was returned to room temperature, 75 µl of distilled water was added to the mixture to disperse it. The fluorescent intensity of the fluorescent substance produced by the reaction between Monochlorobimane and glutathione was measured at 355 nm/460 nm to observe a change in intracellular glutathione content. In this manner, a fraction exhibiting a dose dependent and strong intracellular glutathione increasing activity was identified.

This fraction was again subjected to reverse-phase high-performance liquid chromatography under the same conditions performed above. As a result, it was confirmed that the activity represents a single peak. In order to determine the structure, nuclear magnetic resonance (proton-NMR) analysis and mass analysis (FAB-MS) were performed. Based on the mass analysis data of "(M+H)⁺ being 597.2 and the confirmation of H-NMR signals, it was found that the main substance of the active ingredient of the present invention was rubrofusarin 6-O-β-gentiobioside, as described in Kitanaka & Takido (1988) Chem. Pharm, Bull, 36, 3980; and Hatanaka et al. (1999) ibid., 47, 1121.

### Example 2: Quantitation of rubrofusarin glycoside

It was desired to quantify rubrofusarin glycoside contained in the extract as well as samples at various purification steps in a simple manner. With respect to the rubrofusarin 6-O-β-gentiobioside sample purified and isolated by the method shown in Example 1, preparations each comprising a predetermined weight of the ingredient were prepared based on the molar extinction coefficient (UVλ max nm (log ε)=276(4.72)(Kitanaka & Takido (1988) Chem. Pharm, Bull, 36, 3980) in methanol. Aliquots were taken from the preparations and subjected to reverse-phase high-performance liquid chromatography as described in Example 1 and the heights of the peaks at 280 nm were measured. As a result, it was found that the chromatography is useful for quantitation. Therefore, in this manner, a small amount of rubrofusarin glycoside contained in a sample can be easily determined by chromatography.

### Example 3: Decrease of rubrofusarin glycoside by roasting

Fresh seeds and roasted seeds were broken into pieces respectively. To each of the pulverized samples, a 10-fold volume of 70% (v/v) ethanol was added and extraction was performed for 2 days (at room temperature). Alternatively, to each of the pulverized samples, a 20-fold volume of water was added and extraction was performed under warming at 95°C for 30 minutes. The extract solutions were filtered to obtain filtrates. The weights of the solid matter in the filtrates and the contents of rubrofusarin glycoside in accordance with the method shown in Example 2 were measured. The results are summarized in Table 1.

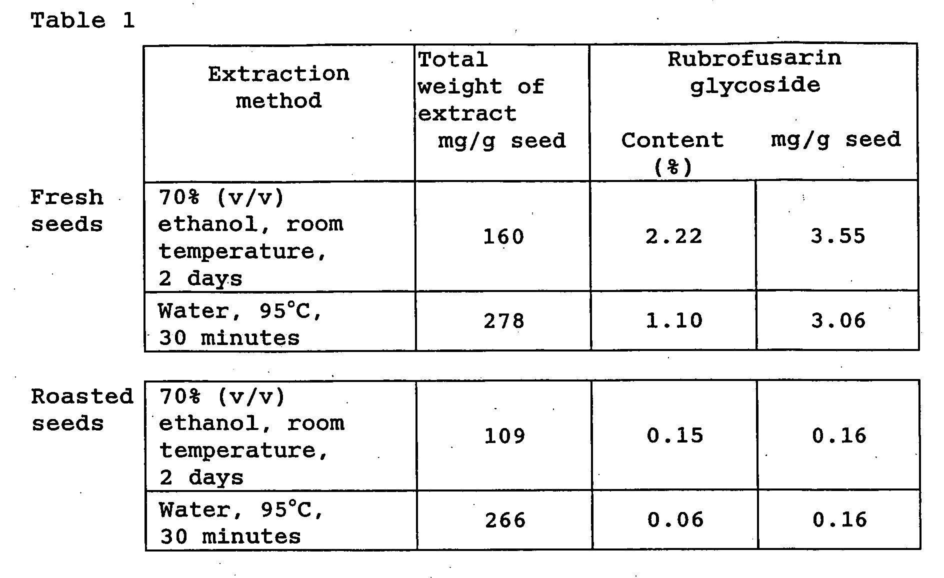

From this Table, it was clear that the content of rubrofusarin glycoside significantly decreased by roasting. In either case of fresh seeds and roasted seeds, the total extraction amount was higher in extraction with water under heating than that with 70% (v/v) ethanol. This is conceivably because a highly viscous polysaccharide was present. As to the extraction amount of rubrofusarin glycoside, more specifically, in terms of extraction rate and absolute extraction amount, 70% (v/v) ethanol extraction was superior. In addition, it was found that the roasted seed extract had a good aroma suitable for teas and beverages, whereas the fresh seed extract had a grassy and fishy odor.

### Example 4: Production of concentrate

To 100 g of non-roasted pulverized Cassia seed, 1.0 L of a 70% (v/v) ethanol solution was added and extraction was performed at room temperature for one whole day and night. The extract was passed through a filter to obtain a filtrate (dry weight: 14.5g, rubrofusarin glycoside: 3.0 wt%). The filtrate was concentrated under vacuum to finally yield 500 ml of a 20% (v/v) ethanol solution, which was then loaded on Diaion HP20 absorbent column (100 ml). Unadsorbed material was washed off with 100 ml of the 20% ethanol solution (total dry-weight of the unadsorbed material: 10.8 g). Elution was performed stepwise with 1000 ml of a 30% (v/v) ethanol solution, and subsequently with 100 ml each of 40%, 50%, 60%, 70%, 80%, and 100%(v/v) ethanol solutions. The dry weight of the eluted material and the weight (wt%) of rubrofusarin glycoside therein are summarized in Table 2.

**Table 2**

| Ethanol conc. for elution (%) | Amount of eluate (column volume) | Dry weight (g) | Rubrofusarin glycoside content (wt%) | Cumulative rubrofusarin glycoside yield(%) |
|---|---|---|---|---|
| 30% | 10 | 2.40 | 9.2 | 50.5 |
| 40% | 1 | 0.23 | 14.3 | 58.2 |
| 50% | 1 | 0.66 | 15.1 | 81.1 |
| 60% | 1 | 0.48 | 10.6 | 92.9 |
| 70% | 1 | 0.25 | 5.1 | 95.9 |
| 80% | 1 | 0.15 | 1.4 | 96.3 |
| 100% | 1 | 0.16 | 0 | 96.3 |

After sufficient washing with a 30% (v/v) ethanol solution under the stated conditions, elution was performed with a 50%(v/v) ethanol solution. As a result, an extraction concentrate (dry weight: 0.89 g) containing 133 mg (14.9 wt%, yield: 30.5%) of rubrofusarin glycoside was obtained from 100 g of Cassia seed.

### Example 5: Time-dependent induction of intracellular glutathione increase

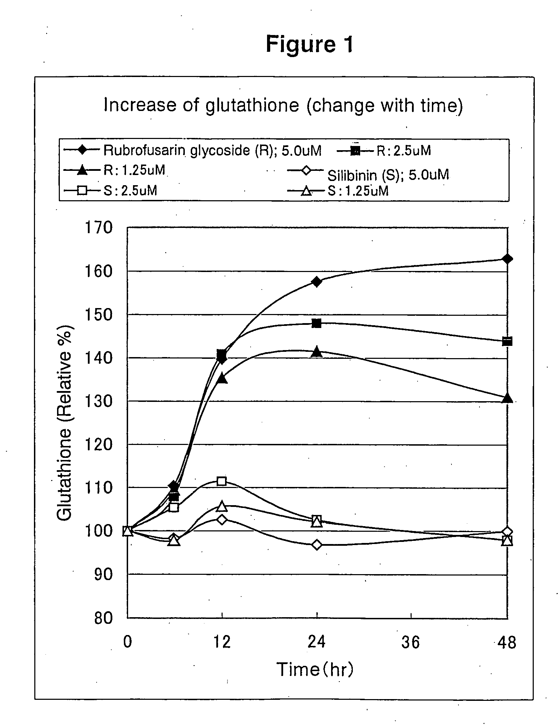

Using human hepatoma HepG2 cells, time-dependent induction effect of rubrofusarin glycoside upon intracellular glutathione level was investigated. The procedure was similar to that of Example 1. Briefly, HepG2 cells were dispensed at a density of 2.5 × 10⁴ cells (100 µl) per well of a microplate and allowed to settle thereto, and then, 25 µl of an evaluation sample diluted to a predetermined concentration in RPMI1640 medium was added. The cells were cultured for 0, 6, 12, 24, and 48 hours. As the evaluation sample, use was made of the concentrate (containing 10.2% (w/w) rubrofusarin gentiobioside) of the present invention prepared in accordance with the method shown in Example 4. As a control, a sample containing silibinin (purchased from Sigma Chemical), a major active ingredient of sirimalin (Planta Med. 55, 420, 1989), which has been reported to increase tissue glutathione level in rats was used to observe change in intracellular glutathione level (Figure 1). In this figure, all data points are expressed by relative values based on the negative control containing no active agent being 100%. To make comparison easier, the concentration of silibinin is expressed in terms of concentration of rubrofusarin glycoside (rubrofusarin gentiobioside) contained in the concentrate (final concentration: 1.25, 2.5, 5.0 µM). As is apparent from Figure 1, 12 hours after the addition of the concentrate of the present invention, it was found that the intracellular glutathione level significantly increased in a dose dependent manner. On the other hand, in silibinin, possibly due to a strong cytotoxicity in the in-vitro cell evaluation system, only a small increase in glutathione level was observed temporarily 12 hours after the addition of silibinin.

### Example 6: Dose dependency of intracellular glutathione increase effect

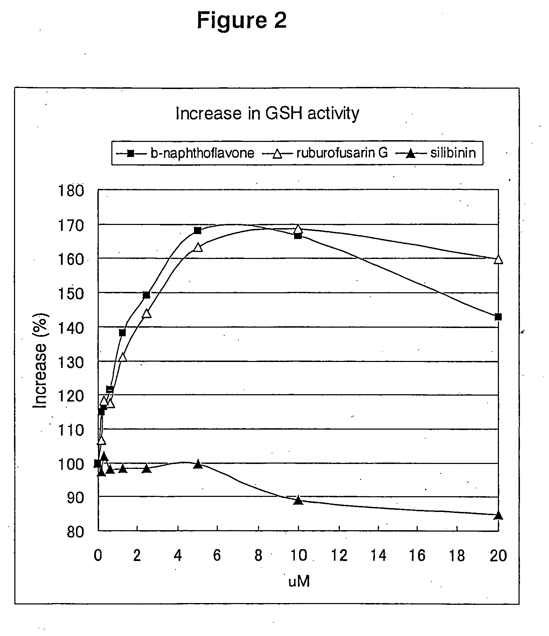

Using human hepatoma HepG2 cells, dose dependency of the effect of rubrofusarin glycoside upon intracellular glutathione level was investigated. The method was similar to that in Example 5. Briefly, HepG2 cells were dispensed at a density of 2.5 × 10⁴ cells (100 µl) per well of a microplate and allowed to settle thereon, and then, 25 µl of the concentrate subjected to 2-fold serial dilution to predetermined concentrations in RPMI1640 medium was added to give a final concentration from 0 to 20 µM in terms of the active ingredient, rubrofusarin gentiobioside. After culturing for an additional 48 hours, the intracellular glutathione level was measured to be compared with that of the cells containing no test-compound or that of the cells containing comparative compound, β-naphthoflavone (purchased from Nacalai-tesque) or silibinin (purchased from Sigma Chemical). The results are shown in Figure 2. Note that β-naphthoflavone is known as a compound which increases the activity of γ-glutamylcysteine synthetase, the rate-determining enzyme of the glutathione biosynthesis system, and intracellular glutathione level in a dose dependent manner, based on cellular level analysis similar to that of this example(J. Biol. Chem. 272, 7445, 1997). As is apparent from these, it was demonstrated that the glutathione increasing activity of the active ingredient of the present invention, rubrofusarin glycoside, was equivalent to that of β-naphthoflavone; however, its cytotoxicity in a high-concentration range was lower. Glutathione increasing activity of silibinin was not observed due to the influence of cytotoxicity.

### Example 7: Protective effect against peroxides

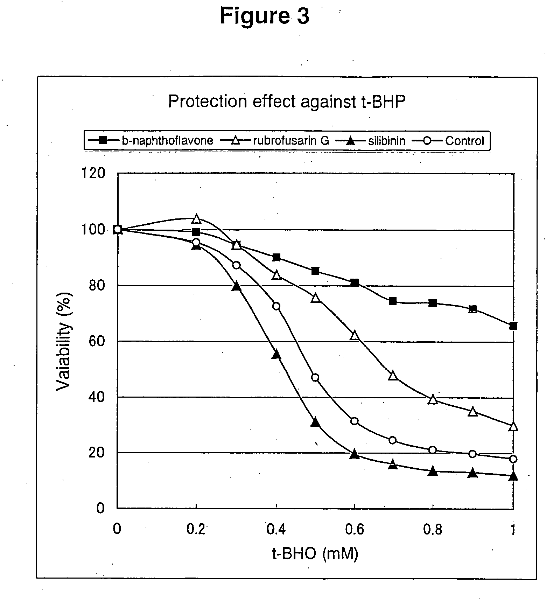

Using human hepatoma HepG2 cells, whether the cells exhibit resistance to peroxides in the presence of rubrofusarin glycoside was investigated. The method was similar to that in Example 5. Briefly, HepG2 cells were dispensed at a density of 2.5 × 10⁴ cells (100 µl) per well of a microplate and allowed to settle thereto, and then, 25 µl of the concentrate diluted in RPMI1640 medium to a final concentration of 10 µM in terms of rubrofusarin gentiobioside was added. After culturing was performed for a further 48 hours, a peroxide, t-butyl hydroperoxide (t-BHP) was added so as to give a final concentration of 0 to 1 mM. The rate of surviving cells after 3 hours relative to that of the cells containing no test compound was compared with those containing comparative compounds, β-naphthoflavone (6 µM) and silibinin (10 µM). The results are shown in Figure 3. The rate of surviving cells was checked by WST-1 staining and calculated based on a control not containing t-BHP as being 100%. As shown in Figure 3, although the protective effect against t-BHP was somewhat lower than that of β-naphthoflavone, it was shown that the active ingredient of the present invention, rubrofusarin glycoside was able to mitigate the cytotoxicity of peroxides in the in vivo t-butyl hydroperoxide model by increasing the level of glutathione, which is a substrate for glutathione peroxidase.

### Example 8: Synergistic effect with a precursor

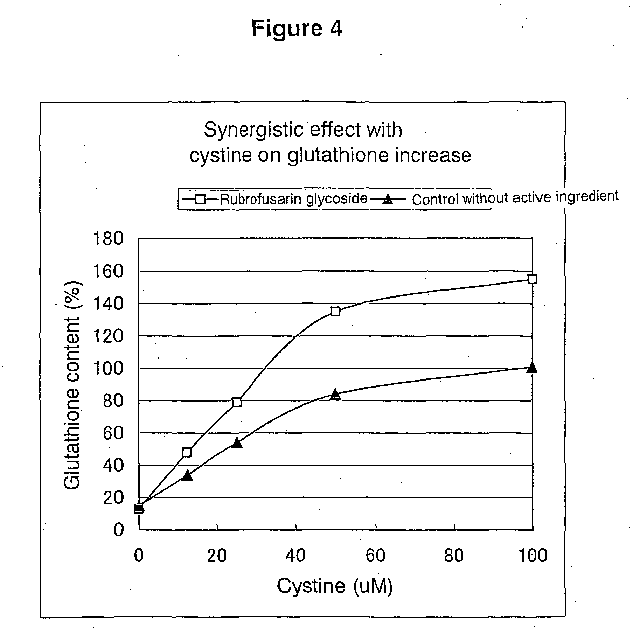

Using human hepatoma HepG2 cells, the synergistic effect between rubrofusarin glycoside having the intracellular glutathione increasing activity and a precursor for synthesizing glutathione in vivo was investigated. The method was similar to that of Example 1 except that D-MEM medium (supplemented with 10% serum) containing no L-cystine was prepared, since RPMI 1649 medium containing a sufficient amount of L-cystine was not suitable for the purpose of this Example. Briefly, HepG2 cells were dispensed at a density of 2.5 × 10⁴ cells (100 µl) per well of a microplate and allowed to settle thereto, and then, the medium was exchanged with the D-MEM medium (100 µl) containing no L-cystine. A diluted concentrate containing rubrofusarin glycoside at a final concentration of 10 µM in L-cystine free D-MEM medium, and cystine solutions of predetermined concentrations were prepared. An aliquot of 25 µl was taken from each of them and added to wells, and culturing was continued for 40 hours. Thereafter, intracellular glutathione levels were measured. As is apparent from the results shown in Figure 4, intracellular glutathione level increased as the concentration of L-cystine added increased. However, no increase in glutathione level was observed by the concentrate of the present invention in the absence of the precursor, L-cystine. However, the co-existence of L-cystine, resulted in a higher glutathione level than that obtained by L-cystine alone. These results indicate that rubrofusarin glycoside having a glutathione increasing activity acted synergistically in cooperation with the glutathione precursor, thereby to increase intracellular glutathione level.

### Example 9: Suppression of restraint stress/organ glutathione level

Glutathione levels in organs are decreased as a result of restraint stress. Whether or not rubrofusarin glycoside has any effect in preventing or lessening such a decrease was investigated. As test animals, 6 week old C57BL/6 male mice, comprising 6 mice in a group, were used. They were given an oral suspension of the concentrate mentioned above in 0.5% Tween 80 at a dose of 200 mg/kg (corresponding to 12 mg/kg in terms of rubrofusarin glycoside) once a day for 8 days. The mice were then fixed on a restraint net for 8 hours to subject them to restraint stress. To a non-restraint control group (n=6), and a restraint control group (n=6), a 0.5% Tween 80 aqueous solution was administered compulsively under the same conditions, and the restraint control group was subjected to the same stress mentioned above. Immediately after the mice were released from restraint, the liver, and brain tissue except for the cerebellum were excised. The contents of glutathione and lipid peroxide (thiobarbituric acid reactive substance: TBARS) of each organ were measured. More specifically, after excision, the organs were stored at -80°C. An ice cold homogenization solution (154 mM KCl, 5 mM DTPA, 10 mM KPi buffer, pH 6.8) was added to each of the organs in a 9-fold volume to weight ratio. Homogenates were prepared by Physcotron (Microtec Co., Ltd.) and TBARS values were calculated in accordance with the method of Ohkawa et. al. (Anal. Biochem. 95. 351-358, 1979). Further, an acid buffer (40 mM HCl, 10 mM DTPA, 20 mM ascorbic acid, 10%TCA) was added to each homogenate in an equal amount and protein was removed by centrifugation. The glutathione content was determined in accordance with the method of Senft et. al. (Anal. Biochem. 280, 80-86, 2000).

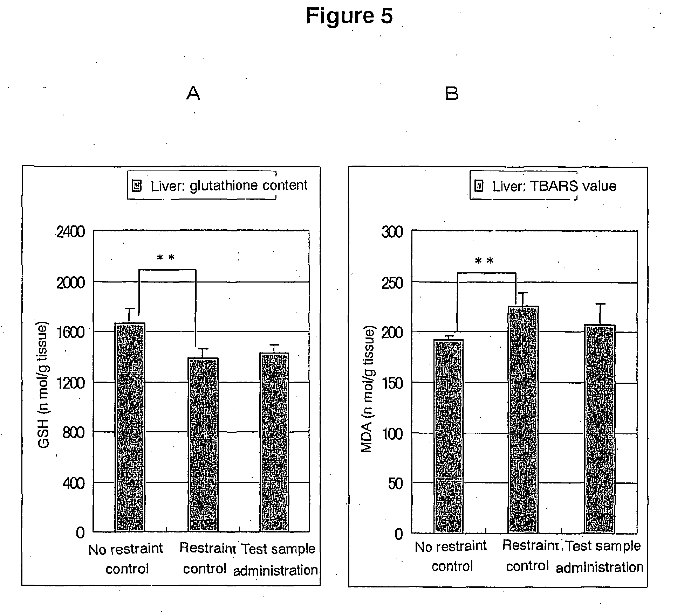

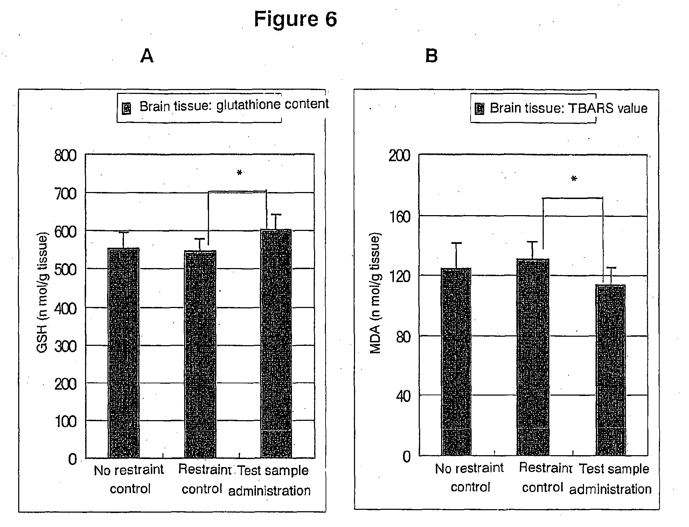

The results in Figures 5 and 6 show that when restraint stress was imparted, glutathione contents of both the liver and brain tissue decreased; at the same time, the TBARS values showing the content of lipid peroxide increased. In contrast, by administering a composition of the present invention containing rubrofusarin glycoside in advance, these values tended to improve towards normal in the liver, and a significant improvement (p<0.05) was observed in brain tissue.

### Example 10: Tablet composition containing the concentrate

After the components shown in Table 3 were uniformly mixed, the mixture was compression-molded into tablets of 300 mg by using a tablet machine. The extraction concentrate used was prepared so as to contain 10 wt% of rubrofusarin glycoside in accordance with the method of Example 4. As a whey peptide, LE80GF manufactured by DMV Japan was used. The standard dose amount per day is 6 tablets each containing 300 mg. In this case, tablets 1, 2 and 3 can supply rubrofusarin glycoside in amounts of 90, 40, 20 mg and L-cysteine/L-methionine in amounts of 0 mg/0 mg, 20 mg/13 mg, and 32 mg/45 mg.

**Table 3**

| | Tablet 1 | Tablet 2 | Tablet 3 |
|---|---|---|---|
| Component | Mixing amount (%) | Mixing amount (%) | Mixing amount (%) |
| Extraction concentrate | 50.0 | 22.2 | 11.1 |
| Whey peptide | | 34.7 | 55.6 |
| L-methionine | | | 1.4 |
| Vitamin C | | 5.6 | 11.1 |
| Cellulose | 6.0 | 20.4 | 11.6 |
| Lactose | 19.0 | 2.1 | |
| Reducing maltose | 16.0 | | |
| Cornstarch | 7.0 | 13.0 | 7.3 |
| Sucrose fatty acid ester | 2.0 | 2.0 | 2.0 |

### Example 11: Beverage composition containing the concentrate

A beverage was prepared by mixing and dissolving 2.5 g of the extraction concentrate containing rubrofusarin glycoside in an amount of 10 wt% and manufactured in accordance with the method according to Example 4, 17.7 g of whey peptide (WE80B manufactured by DMV Japan), 1.2 g of sodium ascorbate, and 26.5 g of sucrose, 2.1 g of citric acid, and 950 g of dissolving water, dispensing the mixture into brown bottles at 120 ml, and subjecting the bottles to retort sterilization (121°C, 15 minutes). It is possible to supply 30 mg of rubrofusarin glycoside and 34 mg of L-cysteine to the body by consuming a single bottle of the beverage.

### ADVANTAGE OF THE INVENTION

As described above, it is possible to maintain/increase glutathione level in cells/tissues by taking the concentrate of the present invention containing rubrofusarin glycoside, thereby preventing or mitigating oxidative stress involved in life-style diseases and their prodromal states, and dysfunction of the brain.

## Claims

1. An oral composition for maintaining or increasing the intracellular glutathione level, wherein the composition contains at least one rubrofusarin glycoside in an amount effective for increasing the intracellular glutathione level.

2. The composition according to claim 1, wherein the rubrofusarin glycoside is extracted from a plant containing rubrofusarin glycosides.

3. The composition according to claim 2, wherein the plant containing rubrofusarin glycoside is Cassia seed or Senna.

4. The composition according to any one of claims 1 to 3, wherein all or the most part of the rubrofusarin glycoside comprises rubrofusarin gentiobioside.

5. The composition according to any one of claims 1 to 4, wherein the content of rubrofusarin glycoside is 0.5 wt% to 30 wt% on solid basis.

6. The composition according to any one of claims 1 to 5, wherein the composition is a food or beverage containing rubrofusarin glycoside.

7. The composition according to any one of claims 1 to 5, wherein the composition is a health food containing rubrofusarin glycoside.

8. The composition according to any one of claims 1 to 5, wherein the composition is a pharmaceutical composition containing rubrofusarin glycoside.

9. The composition according to any one of claims 1 to 8, wherein the composition further contains a precursor for intracellular glutathione biosynthesis and optionally a vitamin C component so as to synergistically increase the intracellular glutathione level.

10. The composition according to claim 9, wherein the precursor for intracellular glutathione biosynthesis is selected from the group consisting of egg white, egg membrane, milk whey, yeast protein, vegetable protein such as soy bean, L-cystine, and L-methionine.

11. The composition according to claim 9 or 10, wherein the composition further comprises a precursor for further augmenting the intracellular glutathione biosynthesis in an amount of 0.1 wt% to 10 wt% on solid basis in terms of L-cysteine or L-methionine.

12. An oral composition for maintaining or increasing the intracellular glutathione level, wherein the composition contains an active ingredient obtained by extraction of pulverized Cassia seed or Senna seed with water or a 10 to 100% organic solvent to yield a crude extract, bringing the crude extract into contact with an adsorbent resin, and performing elution with a 10 to 100% organic solvent.

13. The composition according to claim 12, wherein ethanol is used as the organic solvent for the extraction and the elution.

14. The composition according to claim 12 or 13, wherein rubrofusarin glycoside is contained in an amount of 0.5 wt% to 30 wt% on solid basis.

15. The composition according to claim 14, wherein the rubrofusarin glycoside is rubrofusarin gentiobioside.
